# FASCICULE DE BREVET EUROPEEN

(11) **EP 4 479 072 B1**
(45) Date de publication et mention de la délivrance du brevet: **04.03.2026**
(21) Numéro de dépôt: 23708854.7
(22) Date de dépôt: 17.02.2023
(51) Int. Cl.: A61K 36/37, A61P 17/10, A61K 36/61, A61K 47/44, A61Q 19/00, A61K 9/08

(54) **COMPOSITION COMPRENANT UN EXTRAIT DE MYRTE ET UN EXTRAIT DE TRIPTERYGIUM WILFORDII POUR LUTTER CONTRE L'ACNÉ DES ZONES NON LÉSIONNELLES DE LA PEAU ACNÉIQUE**
ZUSAMMENSETZUNG MIT EINEM MYRTEEXTRAKT UND EINEM TRIPTERYGIUM-WILFORDII-EXTRAKT ZUR BEKÄMPFUNG VON AKNE VON NICHTLÄSIONALEN BEREICHEN VON AKNEHALTIGER HAUT
COMPOSITION COMPRISING A MYRTLE EXTRACT AND A TRIPTERYGIUM WILFORDII EXTRACT FOR CONTROLLING ACNE OF NON-LESIONAL AREAS OF ACNEIC SKIN

(30) Priorité: 17.02.2022 FR 2201379
(43) Date de publication de la demande: 25.12.2024
(73) Titulaire: Pierre Fabre Dermo-Cosmétique, 81500 Lavaur (FR)
(72) Inventeur: MIAS VIGOUROUX, Céline, 81106 CASTRES (FR); DUPLAN, Hélène, 81106 CASTRES (FR); BESSOU-TOUYA, Sandrine, 81106 CASTRES (FR)
(74) Mandataire: Regimbeau
(86) Numéro de dépôt international: PCT/FR2023/050223
(87) Numéro de publication internationale: WO 2023/156745

(56) Documents cités:
- FR-A1- 2 783 425
- FR-A1- 2 992 862
- FR-A1- 3 051 116
- WANG LUN ET AL: "Tripterygium wilfordii Hook F. in the treatment of synovitis, acne, pustulosis, hyperostosis, and osteitis syndrome: a clinical trial", CLINICAL RHEUMATOLOGY, ACTA MEDICA BELGICA, BRUXELLES, BE, vol. 40, no. 6, 3 January 2021 (2021-01-03), pages 2427 - 2438, XP037452834, ISSN: 0770-3198, [retrieved on 20210103], DOI: 10.1007/S10067-020-05562-X

## Description

### DOMAINE TECHNIQUE DE L'INVENTION

La présente invention concerne des compositions cosmétiques ou dermatologiques comprenant un extrait de myrte et un extrait de *Tripterygium wilfordii,* pour leur utilisation dans le domaine de l'acné, et plus particulièrement dans le traitement et/ou la prévention de l'acné des zones non lésionnelles de la peau acnéique (i.e. hors zones lésionnelles avec inflammation visible : papules, pustules et nodules), notamment en agissant directement sur les phylotypes de *C*. *acnes.*

### ETAT DE LA TECHNIQUE

L'acné est une affection cutanée chronique inflammatoire de l'unité pilo-sébacée entrainant une formation de comédons, touchant le visage, la région scapulaire, les bras et les régions intertrigineuses. Elle est la principale cause des dermatoses les plus fréquentes. Il est important de ne pas banaliser cette affection et de la traiter correctement car elle peut avoir des conséquences psychosociales invalidantes notamment du fait de la formation de cicatrices.

L'acné est une pathologie multifactorielle. En effet, divers facteurs de risques peuvent conduire au développement de l'acné ou l'accentuer. Ces facteurs concernent l'imprégnation hormonale et le patrimoine génétique mais aussi des facteurs externes environnementaux, comme la pollution et les UVs. Enfin, le mode de vie peut également influencer l'apparition ou l'amplification de l'acné, à savoir l'alimentation et le stress. Il existe plusieurs formes d'acné. Elle peut d'abord se traduire par l'apparition de simples comédons (points noirs et points blancs). On parle alors d'acné rétentionnelle. Mais ces lésions rétentionnelles peuvent aussi évoluer vers des lésions inflammatoires plus sévères : les papules, pustules et nodules. On parle dans ce cas d'acné inflammatoire. Cependant, il est désormais établi que l'inflammation est présente à tous les stades de l'acné même les plus précoces, c'est-à-dire les stades infracliniques : on parle de micro-inflammation. Classiquement ces deux formes coexistent : il s'agit de l'acné mixte, concernant près de 60% des acnéiques.

L'acné commune ou acné vulgaire, également appelée acné polymorphe juvénile, est la plus courante et comprend quatre stades :
- Le stade 1 correspond à l'acné comédonnienne ou rétentionnelle et est caractérisé par un grand nombre de comédons ouverts et/ou fermés et de microkystes ;
- Le stade 2, ou acné papulo-pustuleuse, est de gravité légère à modérée et est caractérisé par la présence de comédons ouverts et/ou fermés, de microkystes, mais également de papules et pustules rouges. Il affecte principalement le visage et laisse quelques cicatrices ;
- Le stade 3, ou acné papulo-comédonnienne, est plus grave et s'étend au dos, au thorax et aux épaules. Il est accompagné d'un grand nombre de cicatrices ;
- Le stade 4, ou acné nodulo-kystique, présente des nodules et également des pustules pourpres volumineux et douloureux. Il est accompagné de nombreuses cicatrices.

Sous la forme la plus légère, l'acné concerne presque chaque être humain. Sa fréquence est maximale à l'âge de la puberté, mais elle peut se manifester pour la première fois dès l'âge de 7 à 9 ans et jusqu'à des âges dépassant 40 ans. Il est fréquent de souffrir d'acné encore après 25 ans. L'acné touche en outre aussi bien les hommes que les femmes. Au cours de la puberté, sous l'influence des sécrétions hormonales et en particulier des androgènes, mais aussi associé à différents facteurs externes, il est observé une surproduction de sébum appelée hyperséborrhée. Chez des sujets prédisposés à l'acné, cet environnement est propice au développement de la bactérie clé de l'acné, *Cutibacterium acnes* (*C. acnes*) (anciennement appelée *Propionibacterium acnes*)*.* Cette bactérie métabolise les triglycérides cutanés en acides gras irritants *via* des lipases qui agressent la paroi du follicule et du derme environnant, produit également divers enzymes et chimioattractants des cellules phagocytaires de l'immunité, et stimule la production de cytokines pro-inflammatoires par différents types de cellules (sébocytes, kératinocytes, monocytes, notamment) qui aggravent l'inflammation. Cette bactérie joue un rôle pivot dans l'acné, notamment en stimulant la réponse inflammatoire locale (Dagnelie et al., Journal of the European Academy of Dermatology 2019, 33(12), 2340-2348). On a longtemps pensé que la lutte contre cette espèce bactérienne était prioritaire dans le traitement de l'acné. L'usage de divers antimicrobiens topiques sont aujourd'hui encore largement utilisés (peroxyde de benzoyle, érythromycine, triclosan). Mais depuis peu, les chercheurs ont compris que le but n'est pas d'éradiquer *C. acnes* qui est une bactérie commensale, nécessaire à l'homéostasie tissulaire, mais de rétablir un équilibre car l'acné est associée à une perte de la diversité des phylotypes de *C*. *acnes* avec une prédominance du phylotype pro-pathogène IA1. Ce phylotype est pro-pathogène et possède une forte capacité à s'organiser en biofilm conférant une plus forte virulence à cette bactérie. Cette virulence peut être mesurée par la quantification des facteurs de virulence produits par *C*. *acnes.* Contrairement à ce que l'on a longtemps pensé, l'acné n'est pas associée à une multiplication de *C*. *acnes* mais à une modification du ratio des phylotypes et une perte de la richesse en ces phylotypes.

De même, une antibiothérapie systémique plus ou moins prolongée était parfois associée au traitement selon la gravité de l'affection (tétracyclines, doxycycline). Les dermatologues vont aujourd'hui plus se tourner vers des anti-inflammatoires locaux, séborégulateurs.

On a constaté des échecs fréquents à tous ces traitements, dus souvent à une forte proportion de souches résistantes de *C*. *acnes.* Cette résistance peut être la conséquence d'une organisation des populations bactériennes en biofilm. Les biofilms sont des communautés cellulaires bactériennes intégrées dans une matrice extracellulaire excrétée par les microorganismes, composée de polymères de sucres et appelée glycocalyx. Les bactéries sessiles (associées au biofilm) sont phénotypiquement et physiologiquement différentes des bactéries planctoniques (libres). Des études ont confirmé l'aptitude de *C*. *acnes* à former des biofilms aussi bien *in vitro* (Holmberg et al., Clin. Microbiol. Infect. 2009, 15, 787-795) qu'*in vivo* sur des dispositifs médicaux (Craig et al., J. Am. Acad. Dermatol. 2007, 722-724).

Ainsi, il existe toujours un besoin de fournir des traitements plus efficaces de l'acné ne présentant pas d'effets indésirables chez le patient. En particulier, il n'existe pas à ce jour d'agents qui agissent à la fois sur les zones lésionnelles mais également sur les zones infracliniques, c'est-à-dire les zones où l'acné n'est pas encore visible. Le but des futurs traitements n'est donc plus de tuer la bactérie *C*. *acnes* mais de rééquilibrer le microbiote cutané, essentiel pour l'homéostasie de la peau.

### RESUME DE L'INVENTION

La présente invention a pour objet de répondre à ces besoins. En effet, les inventeurs ont mis en évidence, de façon tout à fait inattendue, que des compositions cosmétiques ou dermatologiques comprenant un extrait de myrte et un extrait de *Tripterygium wilfordii* avaient la capacité de diminuer le phylotype IC pro-pathogène, uniquement retrouvé dans les peaux acnéiques, et d'augmenter le phylotype IB associé aux peaux saines. Ces compositions sont donc capables de rééquilibrer les phylotypes de *C*. *acnes* dans des zones non lésionnelles (i.e. hors papules, pustules et nodules) de peaux acnéiques, notamment chez des sujets présentant une acné légère à modérée.

La présente invention a donc pour objet une composition cosmétique ou dermatologique comprenant un extrait de myrte et un extrait de *Tripterygium wilfordii,* en particulier avec au moins un excipient cosmétiquement ou dermatologiquement acceptable, pour son utilisation dans le traitement et/ou la prévention de l'acné de zones non lésionnelles d'une peau acnéique, en particulier du visage.

Divulguée est également l'utilisation d'une composition cosmétique ou dermatologique comprenant un extrait de myrte et un extrait de *Tripterygium wilfordii,* en particulier avec au moins un excipient cosmétiquement ou dermatologiquement acceptable, pour le traitement et/ou la prévention de l'acné de zones non lésionnelles d'une peau acnéique, en particulier du visage.

Divulguée est également l'utilisation d'une composition cosmétique ou dermatologique comprenant un extrait de myrte et un extrait de *Tripterygium wilfordii,* en particulier avec au moins un excipient cosmétiquement ou dermatologiquement acceptable, pour la préparation d'un médicament destiné au traitement et/ou à la prévention de l'acné de zones non lésionnelles d'une peau acnéique, en particulier du visage.

Divulguée est également une méthode de traitement et/ou de prévention de l'acné de zones non lésionnelles d'une peau acnéique, en particulier du visage, comprenant l'administration, notamment l'application topique sur les zones non lésionnelles, à une personne en ayant besoin d'une quantité efficace d'une composition cosmétique ou dermatologique comprenant un extrait de myrte et un extrait de *Tripterygium wilfordii,* en particulier avec au moins un excipient cosmétiquement ou dermatologiquement acceptable.

La présente invention a également pour objet une composition cosmétique ou dermatologique comprenant un extrait de myrte et un extrait de *Tripterygium wilfordii,* en particulier avec au moins un excipient cosmétiquement ou dermatologiquement acceptable, pour son utilisation dans la prévention de l'acné, en particulier du visage.

Divulguée est également l'utilisation d'une composition cosmétique ou dermatologique comprenant un extrait de myrte et un extrait de *Tripterygium wilfordii,* en particulier avec au moins un excipient cosmétiquement ou dermatologiquement acceptable, pour la prévention de l'acné, en particulier du visage.

Divulguée est également l'utilisation d'une composition cosmétique ou dermatologique comprenant un extrait de myrte et un extrait de *Tripterygium wilfordii,* en particulier avec au moins un excipient cosmétiquement ou dermatologiquement acceptable, pour la préparation d'un médicament destiné à la prévention de l'acné, en particulier du visage.

Divulguée est également une méthode de prévention de l'acné, en particulier du visage, comprenant l'administration, notamment l'application topique, à une personne en ayant besoin d'une quantité efficace d'une composition cosmétique ou dermatologique comprenant un extrait de myrte et un extrait de *Tripterygium wilfordii,* en particulier avec au moins un excipient cosmétiquement ou dermatologiquement acceptable.

### DESCRIPTION DETAILLEE DE L'INVENTION

L'invention concerne une composition cosmétique ou dermatologique comprenant un extrait de myrte et un extrait de *Tripterygium wilfordii,* en particulier avec au moins un excipient cosmétiquement ou dermatologiquement acceptable, pour son utilisation dans le traitement et/ou la prévention de l'acné de zones non lésionnelles d'une peau acnéique, en particulier du visage, ou encore dans la prévention de l'acné, en particulier du visage, l'acné étant induite par *C*. *acnes.*

### Extrait de myrte

Le myrte commun, *Myrtus communis* L., est un arbuste de la famille des Myrtaceae. L'extrait de myrte selon la présente invention est réalisé plus particulièrement à partir des feuilles de *Myrtus communis.* De préférence, il s'agit d'une fraction apolaire de parties aériennes, et plus particulièrement des feuilles, de myrte.

Par « faction apolaire », on entend une fraction d'un extrait apolaire, typiquement un extrait apolaire traité par du charbon actif de sorte à éliminer des chlorophylles.

Par « extrait apolaire », on entend un extrait susceptible d'être obtenu à l'aide d'un solvant d'extraction apolaire, tel que par exemple l'acétate d'éthyle, l'acétate d'isopropyle ou un mélange de ceux-ci.

Par « parties aériennes » selon l'invention, on entend les parties de la plante situées au-dessus du sol, par exemple, les feuilles, les tiges, les pétioles et/ou les inflorescences, notamment les feuilles. L'extrait selon la présente invention est susceptible d'être obtenu par extraction à l'aide d'un solvant ou mélange de solvants (appelé solvant d'extraction) choisi parmi :
- les alcools tels que l'éthanol, le méthanol, l'isopropanol,
- les cétones dont l'acétone et la méthyléthylacétone,
- l'hexane,
- le chlorure de méthylène,
- l'éther isopropylique,
- l'acétate d'éthyle ou d'isopropyle,
- et leurs mélanges ;
ou par extraction à l'aide de CO₂ supercritique.

Selon un mode de réalisation, l'extrait de myrte est susceptible d'être obtenu par extraction à l'aide d'acétate d'isopropyle, de préférence à partir des parties aériennes, et en particulier des feuilles, de *Myrtus communis.*

Ainsi, la plante (ou toute partie de celle-ci) est mise en contact avec le solvant d'extraction. Une fois l'extraction effectuée, le mélange plante-solvant d'extraction est filtré de sorte à séparer la phase solvant des résidus de plante (appelés marc). Le marc ainsi obtenu est rincé, typiquement avec le même solvant que le solvant d'extraction, et le solvant de rinçage ainsi obtenu est mélangé avec la phase solvant de sorte à obtenir un jus d'extraction.

Avantageusement, les jus d'extraction, obtenus après filtration et rinçage du marc, sont décolorés par addition de charbon actif, ce qui permet l'élimination des chlorophylles.

L'extrait peut également être stabilisé par addition d'un antioxydant comme par exemple le butylhydroxytoluène ou l'alpha tocophérol, notamment en des quantités comprises entre 0,05 et 1 % en poids d'extrait sec.

L'extrait selon la présente invention présente notamment la caractéristique de comprendre, et notamment d'être riche en, myrtucommulones et acide ursolique. Les myrtucommulones A, B', D, B, isos (isosemimyrtucommulmone) et S (semimyrtucommulone) sont avantageusement présentes dans l'extrait et sont notamment les principales myrtucommulones présentes. Avantageusement, la teneur en myrtucommulones totales dans l'extrait est comprise entre 3 et 10 % en poids d'extrait sec.

La teneur en acide ursolique est comprise entre 10 et 30%, de préférence ≥ 15% en poids d'extrait sec.

Ces molécules portent au moins en partie l'activité revendiquée dans le cadre de la présente invention.

Dans un mode de réalisation particulier de l'invention, l'extrait de myrte sera de préférence tel que décrit dans la demande de brevet EP 1 112 079, ou selon l'exemple 1 de la présente demande. Ce document EP 1 112 079 décrit les propriétés antibactériennes de l'extrait de *Myrtus communis* et ses applications dans des compositions cosmétiques ou dermatologiques.

L'extrait de myrte tel qu'utilisé dans la présente invention a également fait l'objet du brevet FR 2 992 862, son activité anti-biofilm vis-à-vis de *C*. *acnes* y étant décrite.

### Extrait de Tripterygium wilfordii

*Tripterygium wilfordii* est une plante médicinale appartenant à la famille des Celastraceae. Les terpènes sont parmi les composants les plus actifs de la plante et sont localisés majoritairement dans les racines de la plante. On trouve notamment les triterpènes pentacycliques, tels que la Tingénine A (encore appelée Tingénone ou Mayténine), la Tingénine B (encore appelée 22béta-hydroxy-tingénone), le Célastrol, la Pristimérine et la Tripterygone. Ces molécules sont décrites dans la demande de brevet EP 3 4548 75.

Dans un mode de réalisation, l'extrait de *Tripterygium wilfordii* peut être un extrait comprenant, notamment enrichi en, triterpène(s) pentacyclique(s), tels que Tingénine A, Tingénine B et/ou Célastrol, et en particulier un mélange de Tingénine A, Tingénine B et Célastrol. L'extrait peut comprendre éventuellement des composants (dont des composants actifs) issus de la plante autres que des triterpènes pentacycliques.

Par « triterpène pentacyclique » selon l'invention, on entend un triterpène pentacyclique naturellement produit par les cellules de la plante *Tripterygium wilfordii* et notamment le Célastrol de formule Chem. I, la Tingénine A (encore appelée Tingénone ou Mayténine) de formule Chem. II, la Tingénine B (encore appelée 22béta-Hydroxy-tingénone) de formule Chem. III, la Pristimérine de formule Chem. IV, et/ou la Tripterygone de formule Chem. V, de préférence le Célastrol, la Tingénine A et/ou la Tingénine B, notamment le Célastrol ou encore un mélange de Tingénine A, Tingénine B et Célastrol.

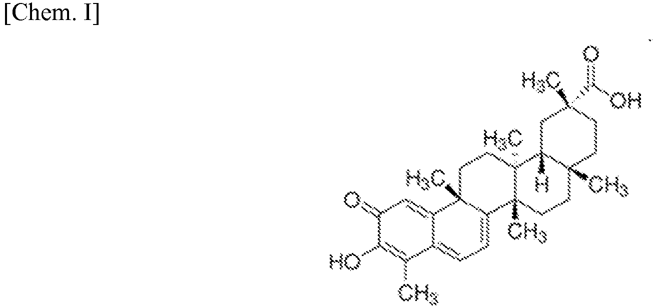

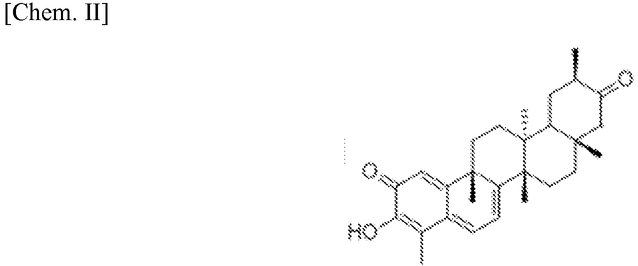

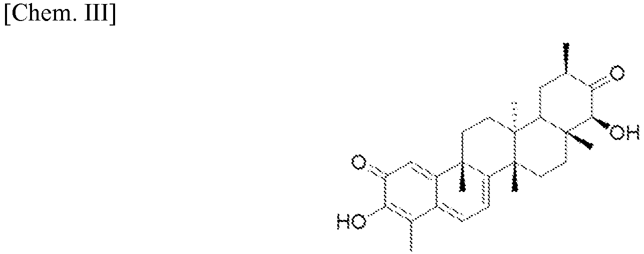

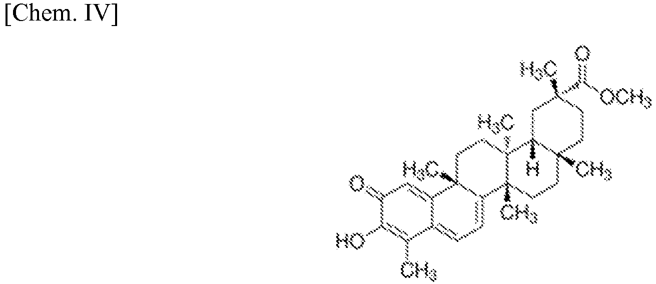

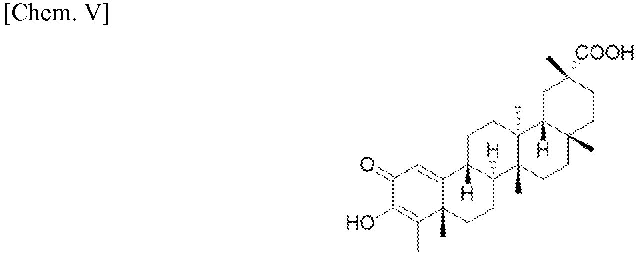

Par « extrait brut », on entend un extrait directement obtenu à partir d'une plante, en l'occurrence *Tripterygium wilfordii.*

Par « extrait enrichi » de *Tripterygium wilfordii,* on entend un extrait de *Tripterygium wilfordii* dans lequel la quantité en triterpène(s) pentacyclique(s), notamment en Tingénine A, Tingénine B et/ou Célastrol, est supérieure à 30% en poids, notamment supérieure à 50% en poids par rapport à la quantité en triterpènes pentacycliques dans un extrait brut sec.

Dans un mode de réalisation, l'extrait, notamment l'extrait enrichi, de *Tripterygium wilfordii* pour une utilisation selon l'invention comprend entre 90% et 100% de triterpène(s) pentacyclique(s) en poids par rapport au poids total de l'extrait sec, notamment de l'extrait enrichi sec.

Les extrait, notamment les extraits bruts ou enrichis, peuvent être obtenus à partir de n'importe quelle partie de la plante *Tripterygium wilfordii,* notamment les racines, les graines ou les parties aériennes.

Alternativement, l'extrait de *Tripterygium wilfordii* peut être obtenu par des cultures de cellules végétales de ces plantes. Dans un tel cas, l'extrait pourra notamment être obtenu à partir du surnageant, de la suspension ou de la biomasse desdites cultures cellulaires, tel que notamment décrit par Coppede et al., Plant Cell Tiss Organ Cult, 2017, 118, 33-43.

Dans un mode de réalisation préféré, l'extrait de *Tripterygium wilfordii* est un extrait comprenant, notamment enrichi en, triterpène(s) pentacyclique(s), et susceptible d'être obtenu selon le procédé suivant :
(i) Une phase de prolifération de cellules de *Tripterygium wilfordii* dans un milieu de prolifération,
(ii) Une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) La préparation d'un extrait comprenant, notamment enrichi en, triterpène(s) pentacyclique(s) à partir de la culture cellulaire obtenue à l'étape (ii).

Par « cellules de *Tripterygium wilfordii »* selon l'invention, on entend les cellules de n'importe quelle partie de la plante : graines, racines, parties aériennes, et notamment des parties aériennes, et plus particulièrement des feuilles.

Par « phase de prolifération de cellules de *Tripterygium wilfordii »,* on entend selon l'invention une phase dans laquelle les cellules de *Tripterygium wilfordii* sont en suspension dans un milieu de prolifération et dans des conditions adaptées à leur prolifération. Ces cellules pourront notamment être obtenues à partir de cals avant leur mise en suspension. Si nécessaire, les suspensions cellulaires pourront être régulièrement réensemencées afin de les maintenir dans des conditions de prolifération.

Par « cal » selon l'invention, on entend un amas de cellules dédifférenciées, également appelées cellules souches ou cellules méristématiques.

Par « environ », on entend dans la présente description que la valeur concernée peut être inférieure ou supérieure de 10%, notamment de 5%, en particulier de 1%, à la valeur indiquée.

L'induction de cals pourra être obtenue par toute méthode connue de l'homme du métier. Les cals selon l'invention pourront notamment être obtenus de la manière décrite ci-après. L'induction de cals à partir d'un explant de tissu de partie de plante, notamment une partie aérienne, telle qu'une feuille, de *Tripterygium wilfordii,* est bien connue de l'homme du métier.

L'induction de cals pourra notamment être réalisée par :
- obtention d'un explant de tissu de la plante, par exemple un morceau de feuille de taille de 1 cm² environ,
- mise en culture de l'explant sur un milieu de prolifération gélosé (par exemple par ajout de 4 à 12 g/L d'agar, par exemple environ 8 g/L d'agar, au milieu de prolifération selon l'invention)
- incubation, notamment à l'obscurité, à une température d'environ 25-30°C, par exemple à environ 27 à 28°C.

Les étapes suivantes : phase de prolifération de cellules, phase d'élicitation et phase de préparation de l'extrait comprenant, notamment enrichi en, triterpènes pentacycliques sont décrites dans le brevet EP 3 4548 75.

### Compositions cosmétiques ou dermatologiques

L'invention vise des compositions cosmétiques ou dermatologiques utiles dans le traitement et/ou la prévention de l'acné de zones non lésionnelles d'une peau acnéique, en particulier du visage, ou encore dans la prévention de l'acné, en particulier du visage.

Par « zones non lésionnelles », on entend, au sens de la présente invention, des zones de la peau ne présentant pas de papules, de pustules et de nodules. On parle encore de stade d'inflammation locale infraclinique.

L'invention vise de préférence des compositions cosmétiques ou dermatologiques selon l'invention se présentant sous une forme adaptée à une application topique.

Les compositions cosmétique ou dermatologique selon l'invention peuvent se présenter ainsi sous les formes qui sont habituellement connues pour une administration topique, c'est-à-dire notamment les lotions, les shampoings, les baumes, les mousses, les gels, les dispersions, les émulsions, les sprays, les sérums, les masques, les crèmes ou les sticks, en particulier avec des excipients permettant notamment, pour certains, une pénétration afin d'améliorer les propriétés et l'accessibilité des principes actifs.

La composition cosmétique ou dermatologique selon l'invention comprendra avantageusement un extrait de myrte qui est une fraction apolaire comprenant des myrtucommulones et l'acide ursolique. En particulier, les myrtucommulones comprennent les myrtucommulones A, B', D, B, l'isosemimyrtucommulone et la semimyrtucommulone.

La composition cosmétique ou dermatologique selon l'invention comprendra avantageusement un extrait de *Tripterygium wilfordii* qui comprend au moins un triterpène pentacyclique tel que défini ci-dessus, et en particulier choisi parmi la Tingénine A, la Tingénine B, le Célastrol, la Pristimérine, la Tripterygone et leurs mélanges, et notamment choisi parmi la Tingénine A, la Tingénine B, le Célastrol et leurs mélanges, et en particulier un mélange de Tingénine A, Tingénine B et Célastrol. L'extrait de *Tripterygium wilfordii* sera avantageusement un extrait de *Tripterygium wilfordii* comprenant, notamment enrichi en, triterpène(s) pentacyclique(s), tels que Tingénine A, Tingénine B et/ou Célastrol, et plus particulièrement un extrait de *Tripterygium wilfordii* comprenant, notamment enrichi en, triterpène(s) pentacyclique(s) susceptible d'être obtenu par le procédé décrit ci-dessus et qui comprend les étapes suivantes :
(i) Une phase de prolifération de cellules de *Tripterygium wilfordii* dans un milieu de prolifération,
(ii) Une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) La préparation d'un extrait comprenant, notamment enrichi en, triterpènes pentacycliques à partir de la culture cellulaire obtenue à l'étape (ii).

La composition cosmétique ou dermatologique selon l'invention comprendra notamment entre 0,01 et 1% d'un extrait de *Tripterygium wilfordii* en poids d'extrait sec par rapport au poids de la composition totale, notamment entre 0,05 et 0,8%, notamment entre 0,1 et 0,5%, notamment entre 0,2 et 0,4% en poids d'extrait sec par rapport au poids de la composition totale. Selon un mode préféré de réalisation, la composition cosmétique ou dermatologique selon l'invention comprend environ 0,3% d'un extrait de *Tripterygium wilfordii* en poids d'extrait sec par rapport au poids de la composition totale.

L'extrait de *Tripterygium wilfordii* comprendra avantageusement 90% ou plus de triterpène(s) pentacyclique(s) en poids par rapport au poids total de l'extrait sec. Dans un mode de réalisation avantageux, l'extrait de *Tripterygium wilfordii* comprendra entre 1 et 20% de Tingénine A en poids par rapport au poids total de l'extrait sec, entre 1 et 20% de Tingénine B en poids par rapport au poids total de l'extrait et au moins 60% de Célastrol en poids par rapport au poids total de l'extrait sec.

La composition cosmétique ou dermatologique selon l'invention comprendra avantageusement entre 0,01 à 1% d'un extrait de myrte en poids d'extrait sec par rapport au poids de la composition totale, notamment entre 0,05 et 0,8%, notamment entre 0,08 et 0,4%, notamment entre 0,08 et 0,2% en poids d'extrait sec par rapport au poids de la composition totale. Selon un mode préféré de réalisation, la composition cosmétique ou dermatologique selon l'invention comprend environ 0,1% d'un extrait de myrte en poids d'extrait sec par rapport au poids de la composition totale. L'extrait de myrte comprendra avantageusement entre 3 et 10% de myrtucommulones en poids par rapport au poids total de l'extrait sec et/ou entre 10 et 30% d'acide ursolique en poids par rapport au poids total de l'extrait sec.

Les exemples qui suivent illustrent l'invention sans en limiter la portée.

### DESCRIPTION DES FIGURES

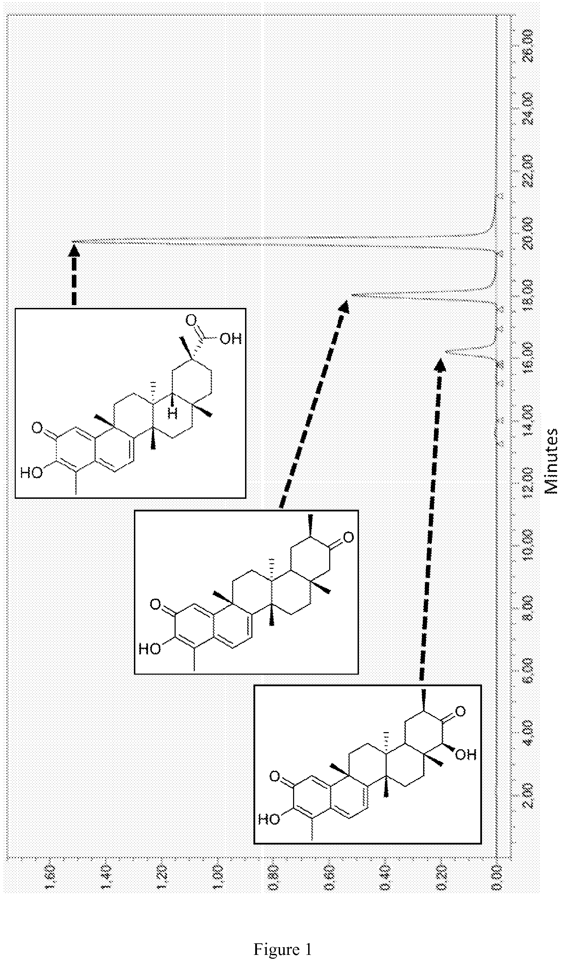

La figure 1 représente le chromatogramme de l'HPLC obtenu pour l'extrait « CCV » de *Tripterygium wilfordii* selon l'exemple 2 contenant les triterpènes pentacycliques suivants : Célastrol, Tingénine A et Tingénine B.

### EXEMPLES

### Exemple 1 : Préparation d'un extrait de myrte

1 kg de feuilles de myrte broyées sont extraites par 5 volumes d'acétate d'isopropyle sous agitation à reflux pendant 1 heure. Après filtration et rinçage du marc, les jus d'extraction sont décolorés par addition de charbon actif. Après filtration, le filtrat décoloré est concentré jusqu'à 2 litres puis séché sur éthanol jusqu'à l'élimination de l'acétate d'isopropyle. La phase aqueuse obtenue est ensuite désodorisée par traitement thermique, puis séchée par lyophylisation.

1 kg de feuilles de myrte permet d'obtenir 25 g environ d'extrait sec de myrte. Celui-ci renferme 7% de myrtucommulones et 25% d'acide ursolique.

### Exemple 2 : Obtention d'un extrait de culture de cellules végétales (CCV) de Tripterygium wilfordii enrichi en triterpènes pentacycliques

On réalise une culture dans un réacteur de type Wave (volume 5 L) de Sartorius Stedim Biotech (Allemagne). On inocule le réacteur avec une suspension de cellules de *Tripterygium wilfordii* issus d'un Erlenmeyer. Le milieu de prolifération a, par exemple, la composition indiquée ci-dessous :
Macroéléments : NH₄NO₃ à 1650 mg/L, KNO₃ à 2500 mg/L, CaCl₂.2H₂O à 440 mg/L, MgSO₄.7H₂O à 370 mg/L, KH₂PO₄ à 130 mg/L ;
Microéléments : KI à 0,41 mg/L, H₃BO₃ à 6,2 mg/L, MnSO₄.4H₂O à 22,3 mg/L, ZnSO₄.H₂O à 7,5 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 19,85 mg/L, Na₂EDTA.2H₂O à 26,64 mg/L ;
Vitamines : myo-inositol à 50 mg/L, acide nicotinique à 0,25 mg/L, pyridoxine-HCl à 0,25 mg/L, thiamine-HCl à 0,25 mg/L ;
Source de carbone : saccharose à 30 g/L ;
Hormones végétales : acide NAA à 0,35 mg/L, acide 2,4 D à 0,575 mg/L, kinétine à 0,083 mg/L. Le pH du milieu est ajusté à pH 6 ± 0,5 (par ajout de KOH, 1M) avant un traitement de stérilisation approprié, par exemple autoclave à 121°C pour une durée minimale de 20 minutes ou par filtration stérilisante sur 0,2 µm.

Sous agitation en continu, après avoir atteint le maximum en biomasse environ 17 jours après, on procède à l'élicitation. Le cocktail d'élicitation est ensuite ajouté dans l'Erlenmeyer au milieu de prolifération à l'aide de solutions mères réalisées dans du diméthylsulfoxyde. La composition du cocktail éliciteur permet d'obtenir les concentrations suivantes dans le milieu d'élicitation (+ cellules) : pyruvate de sodium 1,5 g/L, pyrophosphate de potassium 0,44 g/L, 2iP 0,0004 g/L, jasmonate de méthyle 0,036 g/L et chitine 2 g/L. On arrête la culture après 15 jours d'élicitation. La majorité de la biomasse est récupérée par filtration de la suspension cellulaire avec un filtre nylon (20-50µm). A partir de 5 L de suspension, on récupère environ 1925 g de biomasse. Cette biomasse est extraite avec de l'acétate d'éthyle (ou encore de l'acétate d'isopropyle) en proportion 2 : 1 (Volume : Poids) par rapport au poids de biomasse (ici 3850 mL de solvant pour 1925 g de biomasse). Le mélange biomasse/solvant est alors soumis à une extraction physique, par sonication ou par broyage. La phase organique est alors récupérée après macération sous agitation. L'ajout du solvant (suivi d'une macération sous agitation et de la récupération de la phase organique) est répété deux fois. On a concentré le solvant sous vide, puis solubilisé le concentrat dans du pentylène glycol, et mis le mélange sous vide afin d'éliminer le solvant organique résiduel. On obtient alors une solution nommée « extrait CCV » contenant les triterpènes pentacycliques suivants : Célastrol, Tingénine A et Tingénine B, comme déterminé par le chromatogramme de l'HPLC présenté en Figure 1.

Conditions chromatographie HPLC : appareil de chromatographie liquide Alliance (Waters 2695 version 2.03) ; Colonne Sunfire C18, 100Å, 5 µm (4.6 mm X 150 mm) ; gradient de solvants eau/acétonitrile, débit 3 ml/min ; détection des triterpènes à λ 460 nm.

### Exemple 3 : Formulation d'une composition utile dans le cadre de la présente invention

La formulation A sous forme de crème présente la composition décrite dans le Tableau 1 ci-dessous.

**[Tableau 1]**

| Composants | Quantité (% en poids) |
|---|---|
| Eau | 65,47 |
| Glycérine 99,5% | 5,00 |
| Pentylène glycol | 3,00 |
| Caprylic/Capric Triglycérides 45-55 | 8,00 |
| Dicaprylyl carbonate | 5,00 |
| Benzoique acide | 0,20 |
| Cétéaryl alcool / cétéaryl mélange | 4,00 |
| Polyméthyl méthacrylate | 3,00 |
| Polyacrylate-13/Polyisobutène/Polysorbate 20 | 1,20 |
| Hydroxyde de sodium | 0,03 |
| Stéaryl glycyrrhénate | 0,10 |
| Laurate glycéryl | 0,60 |
| Niacinamide | 4,00 |
| Extrait de *Tripterygium wilfordii* | 0,30 |
| Extrait de feuille de Myrtus communis | 0,10 |

### Exemple 4 : Etude clinique

La pathogenèse de l'acné implique une interaction de quatre facteurs principaux : la surproduction de sébum, l'hyperprolifération des kératinocytes, la modification du microbiote cutané, impliquant notamment la bactérie *Cutibacterium acnes* (*C. acnes*)*,* et l'induction d'une inflammation locale. *C. acnes,* l'espèce bactérienne la plus représentée dans la zone sébacée de la peau, jouerait un rôle majeur dans la pathogenèse de l'acné (Dréno et al. Am.J. Clin. Dermatol. 2020, 21 (Suppl 1) : 18-24). Il est intéressant de noter que des études récentes ont démontré que l'acné résulte d'une perte spécifique de la diversité des phylotypes de *C*. *acnes,* plutôt que d'une hyperprolifération de C. *acnes* (Pécastaings et al. J. Eur. Acad. Dermatol. Venereol. 2018, 32 (Suppl 2) : 15-23). En effet, une prédominance du phylotype IA a été observée. Ce phylotype a été décrit comme ayant une activité plus virulente dans la peau acnéique que dans la peau non-acnéique, *via* sa capacité plus grande à former des biofilms. Une étude récente a montré que, dans les lésions inflammatoires de l'acné, la proportion du phylotype IA de *C*. *acnes* augmente alors que les phylotypes IB et II diminuent (Fourniere et al. Microorganisms 2020, 8(11) :1752). De manière intéressante, les phylotypes IB et II, majoritairement exprimés dans la peau saine, n'altèrent pas l'intégrité des tissus et ne sont pas pro-pathogènes dans le contexte de l'acné (Laclaverie et al Exp. Dermatol. 2021, 30(3) : 347-357). Enfin, le phylotype IC est spécifique de la peau acnéique, aucun phylotype IC n'est détectable sur une peau normale/saine (McDowell et al PloS One 2013, 13 ; 8(9) e70897). Sur la base de la formation de biofilms (directement associés à la virulence des bactéries), le phylotype IA présente la formation de biofilms la plus élevée, suivi directement par les phylotypes IC et II, tandis que les phylotypes IB et III, phylotypes présents uniquement sur les peaux saines, présentent la formation de biofilms la plus faible (Kuehnast et al Int. J. Med. Microbiol. 2018, 308(8) : 1027-1035).

L'étude se découpe en deux parties :
- Evaluer l'efficacité à long terme (56 jours) de l'application de la formulation A selon l'exemple 3 chez 41 patients présentant de l'acné faciale jugée légère à modérée ;
- Estimer l'évolution de la flore microbienne, en particulier de *C*. *acnes,* chez 29 de ces patients (ayant donc suivi le même protocole).

### Première partie de l'étude :

41 patients de 12 à 35 ans sont recrutés, dont 22 adolescents (12-17 ans). Ils présentent une acné légère à modérée avec un total de 15 à 50 lésions acnéiques, 10 à 30 lésions non inflammatoires (comédons ouverts et fermés) et 5 à 20 lésions inflammatoires (papules et pustules) sur le visage, à l'exclusion de la zone du nez. Les sujets présentent au moins un érythème post-inflammatoire ou une hyperpigmentation post-inflammatoire.

Deux applications par jour pendant 56 jours d'une formule A (exemple 3) sont réalisées.

Une première visite d'inclusion à J0 permet une première évaluation. La période d'application du produit se fait à domicile de J1 à J56. Une seconde visite se fait à J8, une fenêtre de ± 1 jour est autorisée pour cette seconde évaluation. Une troisième visite est réalisée à J29, une fenêtre de ± 2 jours est autorisée pour cette troisième évaluation. Une quatrième visite ou visite de fin d'étude est programmée à J57, une fenêtre de ± 2 jours est autorisée pour cette dernière évaluation, à condition que les patients continuent de participer à l'étude.

Différents critères sont évalués :
- Les variations du nombre de lésions acnéiques totales sur le visage entre J0, J8, J29 et J57, le comptage se faisant selon la méthode de Lucky ;
- L'évolution du nombre de lésions rétentionnelles (comédons ouverts et fermés, encore appelés respectivement points noirs et microkystes) et des lésions inflammatoires (papules et pustules) sur le visage entre J0, J8, J29 et J57, le comptage se faisant selon la méthode de Lucky ;
- Le changement de la sévérité de l'acné selon l'Evaluation Globale de l'Acné par l'investigateur entre J0, J8, J29 et J57 ;
- L'évolution globale de l'acné par les sujets à J8, J29 et J57 sur une échelle en 6 points (-1 : aggravation ; 0 : pas de changement ; 1 : légère amélioration ; 2 : amélioration modérée ; 3 : bonne amélioration ; 4 : très bonne amélioration) ;
- L'évolution clinique (par l'investigateur) de la douceur de la peau, sur une échelle de 11 points (0 à 10) entre J0, J8, J29 et J57.

### Résultats

L'évolution des lésions acnéiques est résumée dans le Tableau 2 ci-dessous.

**[Tableau 2]**

| | J0 (moy ± sem) | J8 (moy ± sem) | J29 (moy ± sem) | J57 (moy ± sem) |
|---|---|---|---|---|
| Points noirs | 7,6 ± 1,1 | 6,2 ± 1,1 | 4,1 ± 0,8*** | 4,0 ± 0,8*** |
| Microkystes | 13,6 ± 1.3 | 11,8 ± 1,3* | 10,7 ± 1,2* | 9,1 ± 1,3** |
| LRG | 21,1 ± 1,1 | 18,0 ± 1,3*** | 14,9 ± 1,4*** | 13,1 ± 1,5*** |
| Papules | 5,0 ± 0,3 | 5,1 ± 0,6 | 3,5 ± 0,4** | 3,1 ± 0,5*** |
| Pustules | 3,2 ± 0,5 | 2,4 ± 0,3 | 1,7 ± 0,3*** | 1,7 ± 0,3** |
| LIG | 8,2 ± 0,5 | 7,4 ± 0,6* | 5,2 ± 0,5*** | 4,8 ± 0,7*** |
| **Lésions globales** | **29,3 ± 1,3** | **25,4 ± 1,6***** | **20,0 ± 1,5***** | **17,9 ± 2,0***** |

| | | | | |
|---|---|---|---|---|
| LRG : lésions rétentionnelles globales ; LIG : lésions inflammatoires globales moy ± sem : moyenne ± écart-type *P<0,05 vs J0 ; **P<0,01 vs J0 ; ***P<0,001 vs J0. | | | | |

Sous ces conditions, après 56 jours d'application deux fois par jour, la formule A présente un effet anti-acné significatif sur le nombre des lésions rétentionnelles et inflammatoires (Tableau 2). Cet effet anti-acné apparait dès 8 jours de traitement sur les lésions globales. L'effet protecteur semble progresser tout le long du traitement.

Cette amélioration au cours de l'étude est clairement mise en évidence avec l'évaluation globale de l'acné (EGA), représentée dans le Tableau 3 ci-dessous.

**[Tableau 3]**

| | J0 (moy ± sem) | J8 (moy ± sem) | J29 (moy ± sem) | J57 (moy ± sem) |
|---|---|---|---|---|
| Score EGA | 2,5 ± 0,1 | 2,4 ± 0,1 | 2,1 ± 0,1** | 1,9 ± 0,2*** |

| | | | | |
|---|---|---|---|---|
| **P<0,01 vs J0 ; ***P<0,001 vs J0 | | | | |

A la fin de l'étude 49% des patients présentent une amélioration visible de l'acné.

L'évaluation de l'évolution globale de l'acné par les sujets eux-mêmes (échelle en 6 points) est présentée dans le Tableau 4 ci-dessous, en % des sujets répondant au critère d'évolution de leur acné à J8, J29 et J57.

**[Tableau 4]**

| EGA | J8 | J29 | J57 |
|---|---|---|---|
| -1 : aggravation | 0 | 3 | 8 |
| 0 : pas de changement | 38 | 20 | 17 |
| 1 : légère amélioration | 41 | 32 | 26 |
| 2 : amélioration modérée | 16 | 29 | 31 |
| 3 : bonne amélioration | 5 | 16 | 13 |
| 4 : très bonne amélioration | 0 | 0 | 5 |

Dès 8 jours d'application, 62% des sujets ressentent une amélioration globale (stades 1 à 4) et 5% des patients trouvent que leur peau a eu une bonne amélioration. A la fin de l'étude, 75% des sujets ressentent une amélioration globale (stades 1 à 4) et 18% trouvent que leur peau a eu au moins une bonne amélioration.

Les résultats sur l'estimation de la douceur de la peau (échelle de 0 à 10, 0 : peau très rugueuse ; 10 : peau très douce) au cours de l'étude sont résumés dans le Tableau 5 ci-dessous.

**[Tableau 5]**

| | J0 (moy ± sem) | J8 (moy ± sem) | J29 (moy ± sem) | J57 (moy ± sem) |
|---|---|---|---|---|
| Effet douceur | 4,2 ± 0,2 | 4,8 ± 0,2*** | 5,2 ± 0,3*** | 6,1 ± 0,3*** |

| | | | | |
|---|---|---|---|---|
| ***P<0,001 vs J0 | | | | |

La formule A présente donc un effet lissant par amélioration du score clinique évalué par un dermatologue

Les inventeurs ont clairement démontré que la formule A présente une activité clinique anti-acné remarquable.

### Seconde partie de l'étude :

L'étude est réalisée chez 29 sujets, 15 adolescents (12-17 ans) et 14 adultes. Des prélèvements sur écouvillons sont effectués au niveau du front, sur des zones non lésionnelles, c'est-à-dire que les papules et/ou les pustules sont exclues, on parle de stade d'inflammation locale infracliniques. Les prélèvements sont faits à J1, avant la première application du produit, et au dernier jour de l'étude (J 57).

Il s'agit des mêmes patients (29 parmi les 41 patients de l'étude complète), ils présentent donc une acné légère à modérée et le protocole de traitement est par conséquent le même.

### Séquençage du phylotype de C. acnes

Après extraction de l'ADN des écouvillons, l'analyse du phylotype de *C*. *acnes* est réalisé sur la plateforme Génomique GeT (INRAE) en utilisant le SLST (Singe-Locus Sequence Typing) avec la technologie Illumina Miseq.

À partir de l'ADN extrait, le gène cible de la SLST décrit par Scholz et al. PLOS 1994, 9(8) e104199, est amplifiée par PCR en utilisant des oligonucléotides modifiés. La séquence de chaque fragment d'ADN est ensuite obtenue par séquençage à haut débit. L'analyse des séquences obtenues et leur comparaison avec la liste des séquences de référence issues de la base de données http://medbac.dk/slst/pacnes permettent de déterminer les abondances relatives de chaque phylotype de *C*. *acnes* au sein des différents échantillons. Le séquençage est réalisé sur un séquenceur MiSeq (Illumina). Les séquences présentant 100% d'homologie entre elles sont regroupées en séquences uniques, puis en OTU (unité taxonomique opérationnelle : seuil de 100%) qui seront identifiées ultérieurement. Les OTUs rares (présentes 1 fois avec 1 lecture) sont supprimées. L'analyse bioinformatique des données de séquençage permet d'identifier le phylotype de *C*. *acnes* présent. L'analyse pour l'affiliation phylogénétique jusqu'au niveau du phylotype a été réalisée à l'aide d'outils bioinformatiques pour le traitement de grandes quantités de données de séquence (Mothur). Trois paires de phylotypes ne peuvent être discriminées avec cette méthode : le phylotype D1 est indiscernable du phylotype D2, le phylotype E3 est indiscernable du phylotype E6 et le phylotype F1 est indiscernable du phylotype F11.

Tous les phylotypes SLST obtenus sont regroupés dans les 5 phylotypes suivants :
- IA : séquences A à F
- IC : séquences G
- IB : séquences H
- II : Séquences K
- III : séquences L

L'analyse statistique effectuée sur les données de séquençage donne un indice de diversité et de richesse sur les phylotypes de *C*. *acnes* : IA, IB, IC, II et III.

### Résultats

La distribution des phylotypes de *C*. *acnes* en début d'étude est représentée dans le Tableau 6 suivant.

**[Tableau 6]**

| Phylotypes | Abondance (%) |
|---|---|
| IA | 83,2 |
| IB | 9,0 |
| IC | 1,9 |
| II | 5,7 |
| III | 0,2 |

Comme le montre le Tableau 6, en début d'étude (avant la première application), le phylotype IA est de loin le plus représenté dans les zones non lésionnelles (c'est-à-dire hors papules et pustules) de la peau acnéique du front des volontaires, suivi des phylotypes IB, II, IC et III.

L'analyse statistique de l'évolution des phylotypes entre J0 et J57 est représentée dans le Tableau 7 ci-dessous.

**[Tableau 7]**

| Evolution des phylotypes entre J0 et J57 | Valeur de p | |
|---|---|---|
| IA | 0,69 | NS |
| IB | 0,03 | * |
| IC | 0,01 | ** |
| II | 0,69 | NS |
| III | 0,78 | NS |

| | | |
|---|---|---|
| * : p<0,05 ; **p<0,01 ; NS : non significatif | | |

L'application de la formulation A ne modifie pas l'abondance des phylotypes IA, II et III après 57 jours de traitement. En revanche comme le montre les données du tableau 7, l'application deux fois par jour de la formulation A pendant 57 jours induit une augmentation significative du phylotype IB passant d'une abondance de 8,95 à 9,28% et une diminution significative du phylotype IC, passant d'une abondance de 1,90 à 1,32%.

Une abondance différente des phylotypes de *C*. *acnes* est observée chez les patients acnéiques par rapport au contrôle sain, avec une prédominance du phylotype IA chez les patients acnéiques (Pécastaings et al. 2018).

Dans cette étude, les inventeurs montrent que le phylotype IA est le plus représenté, suivi des phylotypes IB, II, IC et III, comme cela a déjà été décrit sur les zones inflammatoires des patients acnéiques. Mais, ils obtiennent de nouveaux résultats sur l'abondance des phylotypes de *C*. *acnes* sur des zones non lésionnelles de peau acnéique.

En effet, de manière tout à fait inattendue et surprenante, les inventeurs montrent que l'application de la formule A induit une augmentation significative du phylotype non-pathogène IB et réduit de façon significative le phylotype IC spécifique de l'acné. Ces résultats suggèrent donc que la formulation A est efficace dans un contexte d'acné. C'est la première fois que des effets significatifs sur le phylotype IC spécifique de l'acné sont mis en évidence.

Dans cette étude, les inventeurs en utilisant un schéma de typage de séquence à un seul locus pour *C. acnes,* confirment le lien entre la présence de certains phylotypes de *C*. *acnes* et l'acné, et plus particulièrement dans les zones non lésionnelles de sujets avec une acné légère à modérée. De plus les inventeurs démontrent qu'une application prolongée de la formulation A a un impact positif sur les zones non lésionnelles de la peau acnéique en agissant directement sur les phylotypes de *C. acnes,* en diminuant le phylotype IC pro-pathogène et en augmentant le phylotype IB non pathogène. Cette formulation A est donc capable de rééquilibrer les phylotypes de *C*. *acnes* dans des zones non lésionnelles de sujets avec une acné légère à modérée.

L'ensemble de ces résultats démontrent qu'une application prolongée de la formulation A permet non seulement une amélioration de l'acné sur les zones lésionnelles, mais également sur les zones non lésionnelles appelées encore infracliniques, c'est-à-dire les zones où l'acné n'est pas encore visible. Une telle formulation présente donc l'avantage d'être appliquée dans un cadre de prévention d'acné chez des sujets présentant une peau acnéique.

## Revendications

1. Composition cosmétique ou dermatologique comprenant un extrait de myrte et un extrait de *Tripterygium wilfordii,* avec au moins un excipient cosmétiquement acceptable ou dermatologiquement acceptable, pour son utilisation dans le traitement et/ou la prévention de l'acné de zones non lésionnelles d'une peau acnéique.

2. Composition cosmétique ou dermatologique comprenant un extrait de myrte et un extrait de *Tripterygium wilfordii,* avec au moins un excipient cosmétiquement acceptable ou dermatologiquement acceptable, pour son utilisation dans la prévention de l'acné.

3. Composition pour son utilisation selon la revendication 1 ou 2, **caractérisée en ce que** l'extrait de myrte est une fraction apolaire comprenant des myrtucommulones et de l'acide ursolique.

4. Composition pour son utilisation selon la revendication 3, **caractérisée en ce que** les myrtucommulones comprennent les myrtucommulones A, B', D, B, l'isosemimyrtucommulone et la semimyrtucommulone.

5. Composition pour son utilisation selon la revendication 3 ou 4, **caractérisée en ce que** la teneur en myrtucommulones totales est comprise entre 3% et 10% en poids, par rapport au poids total de l'extrait sec de myrte.

6. Composition pour son utilisation selon l'une quelconque des revendications 3 à 5, **caractérisée en ce que** la teneur en acide ursolique est comprise entre 10% et 30% en poids, par rapport au poids total de l'extrait sec de myrte.

7. Composition pour son utilisation selon l'une quelconque des revendications 1 à 6, **caractérisée en ce que** l'extrait de *Tripterygium wilfordii* comprend au moins un triterpène pentacyclique.

8. Composition pour son utilisation selon la revendication 7, **caractérisée en ce que** le au moins un triterpène pentacyclique est choisi parmi la Tingénine A, la Tingénine B, le Célastrol, la Pristimérine, la Tripterygone et leurs mélanges.

9. Composition pour son utilisation selon la revendication 8, **caractérisée en ce que** le au moins un triterpène pentacyclique est choisi parmi la Tingénine A, la Tingénine B, le Célastrol, et leurs mélanges, et de préférence est un mélange de Tingénine A, Tingénine B, et Célastrol.

10. Composition pour son utilisation selon l'une quelconque des revendications 7 à 9, **caractérisée en ce que** l'extrait de *Tripterygium wilfordii* est susceptible d'être obtenu par le procédé suivant :
(i) Une phase de prolifération de cellules de *Tripterygium wilfordii* dans un milieu de prolifération,
(ii) Une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) La préparation d'un extrait comprenant des triterpènes pentacycliques à partir de la culture cellulaire obtenue à l'étape (ii).

11. Composition pour son utilisation selon l'une quelconque des revendications 1 à 10, **caractérisée en ce que** l'extrait de *Tripterygium wilfordii* comprend 90% en poids du au moins un triterpène pentacyclique par rapport au poids total de l'extrait sec.

12. Composition pour son utilisation selon l'une quelconque des revendications 1 à 11, **caractérisée en ce que** l'extrait de *Tripterygium wilfordii* comprend entre 1% et 20% de Tingénine A en poids par rapport au poids total de l'extrait sec, entre 1% et 20% de Tingénine B en poids par rapport au poids de l'extrait sec, et au moins 60% de Célastrol en poids par rapport au poids total de l'extrait sec.

13. Composition pour son utilisation selon l'une quelconque des revendications 1 à 12, **caractérisée en ce qu'**elle comprend 0,01% à 1% d'extrait sec de *Tripterygium wilfordii* en poids par rapport au poids total de la composition.

14. Composition pour son utilisation selon l'une quelconque des revendications 1 à 13, **caractérisée en ce qu'**elle comprend 0,01% à 1% en poids d'un extrait sec de myrte par rapport au poids total de la composition.

15. Composition pour son utilisation selon l'une quelconque des revendications 1 à 14, **caractérisée en ce qu'**elle se présente sous une forme adaptée à une application topique.

## Patentansprüche

1. Kosmetische oder dermatologische Zusammensetzung, die einen Myrtenextrakt und einen *Tripterygium wilfordii-Extrakt* mit mindestens einem kosmetisch akzeptablen oder dermatologisch akzeptablen Hilfsstoff für ihre Verwendung bei der Behandlung und/oder Vorbeugung von Akne in nicht betroffenen Bereichen einer akneanfälligen Haut umfasst.

2. Kosmetische oder dermatologische Zusammensetzung, die einen Myrtenextrakt und einen *Tripterygium wilfordii-Extrakt* mit mindestens einem kosmetisch akzeptablen oder dermatologisch akzeptablen Hilfsstoff für ihre Verwendung bei der Vorbeugung von Akne umfasst.

3. Zusammensetzung für ihre Verwendung nach Anspruch 1 oder 2, **dadurch gekennzeichnet, dass** der Myrtenextrakt eine apolare Fraktion ist, die Myrtucommulone und Ursolsäure umfasst.

4. Zusammensetzung für ihre Verwendung nach Anspruch 3, **dadurch gekennzeichnet, dass** die Myrtucommulone die Myrtucommulone A, B', D, B, Isosemimyrtucommulon und Semimyrtucommulon umfassen.

5. Zusammensetzung für ihre Verwendung nach Anspruch 3 oder 4, **dadurch gekennzeichnet, dass** der Gehalt an Myrtucommulonen insgesamt zwischen 3 und 10 Gew.-%, bezogen auf das Gesamtgewicht des Myrten-Trockenextrakts, liegt.

6. Zusammensetzung für ihre Verwendung nach einem der Ansprüche 3 bis 5, **dadurch gekennzeichnet, dass** der Gehalt an Ursolsäure zwischen 10 und 30 Gew.-%, bezogen auf das Gesamtgewicht des Myrten-Trockenextrakts, liegt.

7. Zusammensetzung für ihre Verwendung nach einem der Ansprüche 1 bis 6, **dadurch gekennzeichnet, dass** der *Tripterygium wilfordii-Extrakt* mindestens ein pentacyclisches Triterpen umfasst.

8. Zusammensetzung für ihre Verwendung nach Anspruch 7, **dadurch gekennzeichnet, dass** das mindestens eine pentazyklische Triterpen aus Tingenin A, Tingenin B, Celastrol, Pristimerin, Tripterygon und deren Mischungen ausgewählt ist.

9. Zusammensetzung für ihre Verwendung nach Anspruch 8, **dadurch gekennzeichnet, dass** das mindestens eine pentazyklische Triterpen aus Tingenin A, Tingenin B, Celastrol und deren Mischungen ausgewählt ist und vorzugsweise eine Mischung aus Tingenin A, Tingenin B und Celastrol ist.

10. Zusammensetzung für ihre Verwendung nach einem der Ansprüche 7 bis 9, **dadurch gekennzeichnet, dass** der *Tripterygium wilfordii-*Extrakt durch das folgende Verfahren erhaltbar ist:
(i) eine Proliferations-Phase von *Tripterygium wilfordii*-Zellen in einem Proliferationsmedium,
(ii) eine Elicitations-Phase durch Zugabe eines Elicitations-Cocktails zu der in Schritt (i) erhaltenen Zellkultur, wobei der Elicitations-Cocktail mindestens einen Elicitor vom Typ einer Monocarbonsäureverbindung und mindestens einen biotischen Elicitor umfasst, und
(iii) die Herstellung eines Extrakts, der pentazyklische Triterpene umfasst, aus der in Schritt (ii) erhaltenen Zellkultur.

11. Zusammensetzung für ihre Verwendung nach einem der Ansprüche 1 bis 10, **dadurch gekennzeichnet, dass** der *Tripterygium wilfordii-*Extrakt 90 Gew.-% des mindestens einen pentazyklischen Triterpens, bezogen auf das Gesamtgewicht des Trockenextrakts, umfasst.

12. Zusammensetzung für ihre Verwendung nach einem der Ansprüche 1 bis 11, **dadurch gekennzeichnet, dass** der *Tripterygium wilfordii-*Extrakt zwischen 1 und 20 Gew.-% Tingenin A, bezogen auf das Gesamtgewicht des Trockenextrakts, zwischen 1 und 20 Gew.-% Tingenin B, bezogen auf das Gewicht des Trockenextrakts, und mindestens 60 Gew.-% Celastrol, bezogen auf das Gesamtgewicht des Trockenextrakts, umfasst.

13. Zusammensetzung für ihre Verwendung nach einem der Ansprüche 1 bis 12, **dadurch gekennzeichnet, dass** sie 0,01 bis 1 Gew.-% *Tripterygium* wilfordii-Trockenextrakt, bezogen auf das Gesamtgewicht der Zusammensetzung, umfasst.

14. Zusammensetzung für ihre Verwendung nach einem der Ansprüche 1 bis 13, **dadurch gekennzeichnet, dass** sie 0,01 bis 1 Gew.-% eines Myrten-Trockenextrakts, bezogen auf das Gesamtgewicht der Zusammensetzung, umfasst.

15. Zusammensetzung für ihre Verwendung nach einem der Ansprüche 1 bis 14, **dadurch gekennzeichnet, dass** sie in einer für eine topische Anwendung geeigneten Form vorliegt.

## Claims

1. Cosmetic or dermatological composition comprising a myrtle extract and a *Tripterygium wilfordii* extract, with at least one cosmetically acceptable or dermatologically acceptable excipient, for its use in the treatment and/or prevention of acne in non-lesional areas of acne-prone skin.

2. Cosmetic or dermatological composition comprising a myrtle extract and a *Tripterygium wilfordii* extract, with at least one cosmetically acceptable or dermatologically acceptable excipient, for its use in the prevention of acne.

3. Composition for its use according to claim 1 or 2, **characterized in that** the myrtle extract is a nonpolar fraction comprising myrtucommulones and ursolic acid.

4. Composition for its use according to claim 3, **characterized in that** the myrtucommulones comprise myrtucommulones A, B', D, B, isosemimyrtucommulone, and semimyrtucommulone.

5. Composition for its use according to claim 3 or 4, **characterized in that** the total myrtucommulone content is comprised between 3% and 10% by weight, relative to the total weight of the dry myrtle extract.

6. Composition for its use according to any one of claims 3 to 5, **characterized in that** the ursolic acid content is comprised between 10% and 30% by weight, relative to the total weight of the dry myrtle extract.

7. Composition for its use according to any one of claims 1 to 6, **characterized in that** the *Tripterygium wilfordii* extract comprises at least one pentacyclic triterpene.

8. Composition for its use according to claim 7, **characterized in that** the at least one pentacyclic triterpene is chosen from Tingenin A, Tingenin B, Celastrol, Pristimerin, Tripterygone, and their mixtures.

9. Composition for its use according to claim 8, **characterized in that** the at least one pentacyclic triterpene is chosen from Tingenin A, Tingenin B, Celastrol, and their mixtures, and preferably is a mixture of Tingenin A, Tingenin B, and Celastrol.

10. Composition for its use according to any one of claims 7 to 9, **characterized in that** the *Tripterygium wilfordii* extract is obtainable by the following process:
(i) A phase of proliferation of *Tripterygium wilfordii* cells in a proliferation medium,
(ii) An elicitation phase by adding an elicitation cocktail to the cell culture obtained at step (i), said elicitation cocktail comprising at least one monocarboxylic compound elicitor and at least one biotic elicitor, and
(iii) The preparation of an extract comprising pentacyclic triterpenes from the cell culture obtained at step (ii).

11. Composition for its use according to any one of claims 1 to 10, **characterized in that** the *Tripterygium wilfordii* extract comprises 90% by weight of the at least one pentacyclic triterpene relative to the total weight of the dry extract.

12. Composition for its use according to any one of claims 1 to 11, **characterized in that** the *Tripterygium wilfordii* extract comprises between 1% and 20% of Tingenin A by weight relative to the total weight of the dry extract, between 1% and 20% of Tingenin B by weight relative to the weight of the dry extract, and at least 60% of Celastrol by weight relative to the total weight of the dry extract.

13. Composition for its use according to any one of claims 1 to 12, **characterized in that** it comprises 0.01% to 1% dry extract of *Tripterygium wilfordii* by weight relative to the total weight of the composition.

14. Composition for its use according to any one of claims 1 to 13, **characterized in that** it comprises 0.01% to 1% by weight of dry extract of myrtle relative to the total weight of the composition.

15. Composition for its use according to any one of claims 1 to 14, **characterized in that** it is in a form suitable for topical application.
